(19) Europäisches Patentamt
European Patent Office
Office européen des brevets

(11) Publication number: **0 165 781**
**A1**

# (12) EUROPEAN PATENT APPLICATION

(21) Application number: 85304236.4

(22) Date of filing: 14.06.85

(51) Int. Cl.[4]: A 61 K 31/415
A 61 K 31/41, A 61 K 31/445
A 61 K 31/495, C 07 D 405/06

(30) Priority: 18.06.84 US 621405

(43) Date of publication of application:
27.12.85 Bulletin 85/52

(84) Designated Contracting States:
BE CH DE FR GB IT LI LU NL SE

(71) Applicant: ELI LILLY AND COMPANY
Lilly Corporate Center
Indianapolis Indiana 46285(US)

(72) Inventor: Hirsch, Kenneth Steven
R.R. 1 Box BC23
New Palestine Indiana 46163(US)

(72) Inventor: Taylor, Harold Mellon
7459 Steinmeier Drive
Indianapolis Indiana 46250(US)

(74) Representative: Tapping, Kenneth George et al,
Erl Wood Manor
Windlesham Surrey, GU20 6PH(GB)

## (54) Aromatase inhibiting N-substituted imidazole derivatives.

(57) Use of certain N-substituted imidazole derivatives in preparing a medicament for inhibiting aromatase or treating or preventing estrogen-dependent disease.

EP 0 165 781 A1

Croydon Printing Company Ltd.

# AROMATASE INHIBITING N-SUBSTITUTED IMIDAZOLE DERIVATIVES

This invention is directed to the use of a compound of the formula

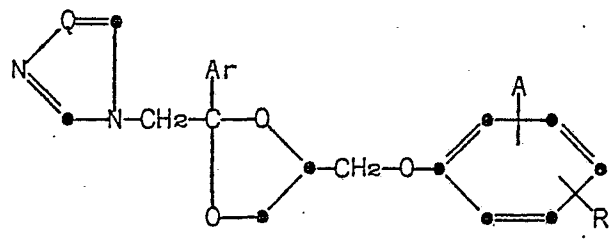

wherein:

Q is CH or N;

Ar is a member selected from the group consisting of phenyl and substituted phenyl, said substituted phenyl being phenyl having from 1 to 3 substituents independently selected from the group consisting of halo, lower alkyl and lower alkyloxy;

the radical A is a member selected from the group consisting of:

(a) an isothiocyanato group -N=C=S;

(b) an amino radical of the formula

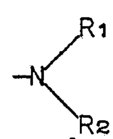

wherein $R_1$ and $R_2$ are each independently selected from the group consisting of hydrogen and lower alkyl;

(c) a radical of the formula

$$-NH-\overset{X}{\overset{\|}{C}}-(Y)_m-R_3$$

wherein X is selected from the group consisting of O and S, Y is selected from the group consisting of O and NH, m is the integer 0 or 1, and $R_3$ is selected from the group consisting of hydrogen, lower alkyl, mono- and dihalo(lower alkyl), phenyl and substituted phenyl, said substituted phenyl having from 1 to 2 substituents independently selected from the group consisting of halo, lower alkyl and lower alkyloxy, provided that:

(i) when said X is S, then said Y is NH and said m is 1, and

(ii) when said Y is O and said m is 1, then said $R_3$ is other than hydrogen; and

(d) a radical of the formula

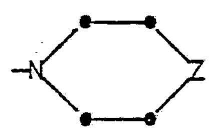

wherein Z is a member selected from the group consisting of a direct bond, $CH_2$, O, and N-$R_4$, wherein $R_4$ is selected from the group consisting of hydrogen, lower alkyl, hydroxy-(lower alkyl), (lower alkyloxy)-lower alkyl, lower alkanoyl, lower alkylsulfonyl, phenylmethylsulfonyl, lower alkyloxycarbonyl, lower alkyloxycarbonylmethyl, phenoxycarbonyl, aminocarbonyl, mono- and di(lower alkyl)aminocarbonylmethyl, amino-

carbonylmethyl, (lower alkyl)aminocarbonylmethyl, (lower alkyl)aminothioxomethyl, (lower alkylthio)thioxomethyl, phenyl, phenylmethyl, benzoyl and substituted benzoyl, said substituted benzoyl being benzoyl having from 1 to 2 substituents independently selected from the group consisting of halo, lower alkyl and lower alkyloxy; and

R is a member selected from the group consisting of hydrogen and nitro, provided that when said R is nitro, then said A is amino, or a pharmaceutically acceptable acid addition salt or stereochemically isomeric form thereof, in preparation of a medicament for inhibiting aromatase or treating or preventing estrogen-dependent disease.

Estrogens are synthesized from androgenic steroids. In the biosynthetic pathway for estrogen formation, aromatization is an essential step. It is generally believed that if the aromatase enzyme could be effectively inhibited, a useful treatment for estrogen dependent disorders could be obtained (see Cancer Research, Vol. 42, Suppl. 8:3261s (1982)).

Several estrogen dependent diseases exist which could be treated with aromatase inhibitors. These include breast cancer, endometriosis, polycystic ovarian disease, benign breast disease, and endometrial cancer. A beneficial effect of antiestrogens in the treatment of breast cancer has been well established (see Br. J. Cancer, 25, 270 (1971)). Two of the known aromatase inhibitors, testolactone and aminoglutethimide, have shown a beneficial effect in treatment of breast cancer. See Cancer Research, supra.

Endometriosis is characterized by an abnormal proliferation of the endometrium of the uterus. Since the endometrium is dependent on estradiol for its growth, an inhibitor of estrogen production should stop the progression of the disease.

Benign breast disease, or often called fibrocystic breast disease, appears to be dependent on ovarian steroids. See Cancer, 49, 2534 (1982). Aromatase inhibitors have not been tried in this disease, but antiestrogens seem to be of benefit. See Obstet. Gynecol., 54, 80 (1979).

Polycystic ovarian disease is one of the most common causes of infertility in women. The disease appears to result from an abnormality in steroid metabolism, and the major form of therapy in this disease is the antiestrogen, clomiphene. See Clin. Endocrinol., 12, 177 (1980).

Ketoconazole [1-acetyl-4-[4-([2-(2,4-dichlorophenyl)-2-(1H-imidazol-1-ylmethyl)-1,3-dioxolan-4-yl]-methoxy)phenyl]piperazine] is one of many orally effective antifungal compounds claimed in U.S. Patent No. 4,335,125. More recently, ketoconazole has been found to inhibit adrenal steroid synthesis by preventing the formation of cholesterol. A. Pont, et al., Annals of Internal Medicine, 97, 370 (1982). Because of this steroidal blockade, it was postulated that a potential beneficial use of ketoconazole would be in the treatment of a variety of disorders, including breast cancer. No disclosure or inference relating to the compound's effect on aromatase or as a specific inhibitor of estrogen synthesis is taught.

By virtue of their ability to inhibit the enzyme aromatase, the compounds of the above formula are useful in the treatment and prevention of estrogen-dependent diseases in mammals.

A preferred group of compounds of this invention are those wherein:

(a) Q is CH,

(b) Ar is mono- or di-halo substituted phenyl, especially where halo is chloro,

(c) R is hydrogen, and

(d) A is 4-(lower alkanoyl)-1-piperazinyl.

The most preferred compound is 1-acetyl-4-[4-([2-(2,4-dichlorophenyl)-2-(1H-imidazol-1-ylmethyl)-1,3-dioxolan-4-yl]methoxy)phenyl]piperazine and its pharmaceutically acceptable salts and stereochemically isomeric forms thereof.

The term "lower alkyl" refers to branched and straight chain aliphatic radicals of one to six carbon atoms such as methyl, ethyl, propyl, isopropyl, butyl, tert-butyl, hexyl, and the like. The term "lower alkanoyl" refers to straight and branched chain alkanoyl radicals of one to six carbon atoms such as formyl, acetyl, propanoyl, butanoyl, hexanoyl, and the like. The term "halo" refers to fluoro, chloro, bromo, and iodo.

The compounds used in this invention and methods of making the compounds are disclosed in U.S. Patents Nos. 4,335,125, 4,144,346 and 4,223,036. The terms used in this application are the same as those used and defined in the above patents. The compounds as disclosed in the patents are described as being useful

as antifungal and antibacterial agents. The patents do not disclose any utility related to the inhibition of aromatase or the treatment of estrogen-dependent diseases.

As will be recognized by those skilled in the art, the compounds of the above formula contain two asymmetric carbon atoms. This invention is not limited to any particular isomer but includes the individual stereoisomers as well as the mixtures of the compounds.

The pharmaceutically acceptable acid addition salts used in this invention include salts derived from inorganic acids such as hydrochloric acid, nitric acid, phosphoric acid, sulfuric acid, hydrobromic acid, hydriodic acid, phosphorous acid and the like, as well as salts derived from organic acids such as aliphatic mono- and di-carboxylic acids, phenyl-substituted alkanoic acids, hydroxy-alkanoic and -alkanedioic acids, aromatic acids, aliphatic and aromatic sulfonic acids, etc. Such pharmaceutically acceptable salts thus include sulfate, pyrosulfate, bisulfate, sulfite, bisulfite, nitrate, phosphate, nonohydrogenphosphate, dihydrogenphosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate,

β-hydroxybutyrate, glycolate, malate, tartrate, methanesulfonate, propanesulfonate, naphthalene-1-sulfonate, naphthalene-2-sulfonate and the like salts. The preferred salts of this invention are those derived from inorganic acids, especially hydrochloric acid.

The compounds used in this invention are useful in preventing or therapeutically treating estrogen-dependent diseases in mammals by virtue of their ability to inhibit the enzyme aromatase. The ability to inhibit aromatase was demonstrated by employing a modification of the isolated rat ovarian microsome method of Brodie et al. in J. Steroid Biochem., 7, 787 (1976). In this test system, ovarian microsomes are obtained from rats treated with pregnant mares serum gonadotropin. Test compounds are added to reaction vials containing 0.1 μM 4-androstene-3,17-dione, 100,000 dpm 1,2[$^3$H]-androstenedione, the microsomes and a NADPH generating system. The concentrations of the inhibitors tested ranged between 0.005 and 10 μM. In this assay, aromatization of androstenedione results in the production of [$^3$H]-$H_2O$ which is isolated by extracting the samples with chloroform and treating the aqueous phase with charcoal to remove the free steroid. Samples are counted in a liquid scintillation spectrometer and the percent inhibition determined by comparing the results with control samples incubated without inhibitor. Potency is determined based on the concentration of inhibitor in μM required to produce a 50% inhibition of enzyme activity ($EC_{50}$) when the concentration of substrate (androstenedione) is 0.1 μM. The $EC_{50}$ of 1-acetyl-4-[4-([2-(2,4-dichlorophenyl)-2-(1H-imidazol-1-ylmethyl)-1,3-dioxolan-4-yl]methoxy)-phenyl]piperazine (ketoconazole) was 1.1 μM.

The compounds may be administered by any number of routes, including the oral, subcutaneous, intramuscular, intravenous, transdermal, and rectal routes. The compounds are usually employed in the form of pharmaceutical compositions. Such compositions are prepared in a manner well known in the pharmaceutical art and comprise at least one active compound of the above formula.

Such pharmaceutical compositions comprise as active ingredient a compound of the above formula associated with a pharmaceutically acceptable carrier. In making the compositions, the active ingredient will usually be mixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the composition can be in the form of tablets, pills, powders, lozenges, sachets, cachets, elixirs, emulsions, solutions, syrups, suspensions, aerosols (as a solid or in a liquid medium), ointments containing for example up to 10% by weight of the active compound, soft and hard gelatin capsules, suppositories, sterile injectable solutions, and sterile packaged powders.

Some examples of suitable carriers, excipients, and diluents include lactose, dextrose, sucrose, sorbitol, mannitol, starches, gum acacia, calcium phosphate, alginates, calcium silicate, microcrystalline

cellulose, polyvinylpyrrolidone, cellulose, tragacanth, gelatin, syrup, methyl cellulose, methyl- and propylhydroxybenzoates, talc, magnesium stearate, water, and mineral oil. The formulations can additionally include lubricating agents, wetting agents, emulsifying and suspending agents, preserving agents, sweetening agents or flavoring agents. The compositions may be formulated so as to provide quick, sustained, or delayed release of the active ingredient after administration to the patient by employing procedures well known in the art.

For oral administration, a compound of this invention can be admixed with carriers and diluents molded into tablets or enclosed in gelatin capsules. The mixtures can alternatively be dissolved in liquids such as ten percent aqueous glucose solution, isotonic saline, sterile water, or the like, and administered intravenously or by injection. Such solutions can, if desired, be lyophilized and stored in a sterile ampoule ready for reconstitution by the addition of sterile water for ready intramuscular injection.

The compositions are preferably formulated in a unit dosage form, each dosage containing from about 1 to about 500 mg, more usually about 5 to about 300 mg, of the active ingredient. The term "unit dosage form" refers to physically discrete units suitable as unitary dosages for human subjects and other mammals, each unit containing a predetermined quantity of active material calculated to produce the desired therapeutic effect, in association with the required pharmaceutical carrier.

The active compounds are effective over a wide dosage range. For example, dosages per day will

normally fall within the range of about 0.05 to about 300 mg/kg of body weight. In the treatment of adult humans, the range of about 0.1 to about 50 mg/kg, in single or divided doses, is preferred. However, it will be understood that the amount of the compound actually administered will be determined by a physician, in the light of the relevant circumstances including the condition to be treated, the choice of compound to be administered, the age, weight, and response of the individual patient, the severity of the patient's symptoms, and the chosen route of administration, and therefore the above dosage ranges are not intended to limit the scope of the invention in any way.

CLAIMS

1. Use of a compound of the formula

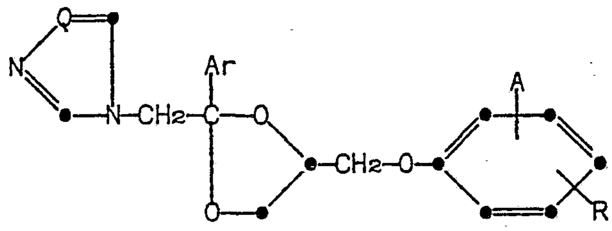

wherein:

Q is CH or N;

Ar is a member selected from the group consisting of phenyl and substituted phenyl, said substituted phenyl being phenyl having from 1 to 3 substituents independently selected from the group consisting of halo, lower alkyl and lower alkyloxy;

the radical A is a member selected from the group consisting of:

(a) an isothiocyanato group -N=C=S;

(b) an amino radical of the formula

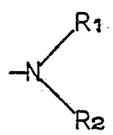

wherein $R_1$ and $R_2$ are each independently selected from the group consisting of hydrogen and lower alkyl;

(c) a radical of the formula

$$-NH-\overset{X}{\overset{\parallel}{C}}-(Y)_m-R_3$$

wherein X is selected from the group consisting of O and S, Y is selected from the group consisting of O and NH, m is the integer 0 or 1, and $R_3$ is selected from the group consisting of hydrogen, lower alkyl, mono- and dihalo(lower alkyl), phenyl and substituted phenyl, said substituted phenyl having from 1 to 2 substituents independently selected from the group consisting of halo, lower alkyl and lower alkyloxy, provided that:

(i) when said X is S, then said Y is NH and said m is 1, and

(ii) when said Y is O and said m is 1, then said $R_3$ is other than hydrogen; and

(d) a radical of the formula

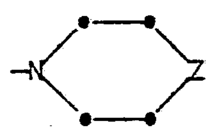

wherein Z is a member selected from the group consisting of a direct bond, $CH_2$, O, and N-$R_4$, wherein $R_4$ is selected from the group consisting of hydrogen, lower alkyl, hydroxy-(lower alkyl), (lower alkyloxy)-lower alkyl, lower alkanoyl, lower alkylsulfonyl, phenylmethylsulfonyl, lower alkyloxycarbonyl, lower alkyloxycarbonylmethyl, phenoxycarbonyl, aminocarbonyl,

mono- and di(lower alkyl)aminocarbonylmethyl, aminocarbonylmethyl, (lower alkyl)aminocarbonylmethyl, (lower alkyl)aminothioxomethyl, (lower alkylthio)thioxomethyl, phenyl, phenylmethyl, benzoyl and substituted benzoyl, said substituted benzoyl being benzoyl having from 1 to 2 substituents independently selected from the group consisting of halo, lower alkyl and lower alkyloxy; and

R is a member selected from the group consisting of hydrogen and nitro, provided that when said R is nitro, then said A is amino, or a pharmaceutically acceptable acid addition salt or stereochemically isomeric form thereof in preparing a medicament for inhibiting aromatase or treating or preventing an estrogen-dependent disease.

2. The use according to claim 1 employing a compound wherein Q is CH.

3. The use according to claim 2 employing a compound wherein Ar is mono- or di-halo substituted phenyl.

4. The use according to claim 3 employing a compound wherein R is hydrogen.

5. The use according to claim 4 employing a compound wherein A is 4-(lower alkanoyl)-1-piperazinyl.

6. The use according to claim 4 employing a compound wherein A is 4-acetyl-1-piperazinyl.

7. The use according to claim 1 employing 1-acetyl-4-[4-([2-(2,4-dichlorophenyl)-2-(1H-imidazol-1-ylmethyl)-1,3-dioxolan-4-yl]methoxy)phenyl]piperazine or a pharmaceutically acceptable salt thereof.

European Patent Office

# EUROPEAN SEARCH REPORT

0165781
Application number

EP 85304236.4

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int Cl 4) |
| D,X | ANNALS OF INTERNAL MEDICINE, vol. 97, July through December 1982, Philadelphia<br>A. PONT et al. "Ketoconazole Blocks Adrenal Steroid Synthesis" pages 370-372<br>* Page 370; abstract; left column; page 372, left column, last passage *<br>-- | 1-7 | A 61 K 31/415<br>A 61 K 31/41<br>A 61 K 31/445<br>A 61 K 31/495<br>C 07 D 405/06 |
| A | US - A - 4 101 664 (J. HEERES)<br>* Claim 4; column 1, lines 21-52 *<br>-- | 1-4 | |
| A | US - A - 4 439 441 (D.W. HALLESY et al.)<br>* Abstract; column 2, lines 17-27, 58-68 *<br>-- | 1-7 | TECHNICAL FIELDS SEARCHED (Int Cl 4) |
| D,A | US - A - 4 144 346 (J. HEERES et al.)<br>* Column 1, line 23 - column 2, line 49; example XLVIII *<br>-- | 1-7 | A 61 K 31/00<br>C 07 D 405/00 |
| D,A | CANCER RESEARCH, vol. 42, no. 8, Supplement, August 1982, pages 3261s-3469s<br>* Pages 3312s-3320s, 3430s-3433s, 3458s-3460s *<br>---- | | |

The present search report has been drawn up for all claims

| Place of search | Date of completion of the search | Examiner |
|---|---|---|
| VIENNA | 12-09-1985 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document